# EUROPÄISCHE PATENTSCHRIFT

(11) **EP 0 695 319 B1**
(45) Veröffentlichungstag und Bekanntmachung des Hinweises auf die Patenterteilung: **02.12.1998**
(21) Anmeldenummer: 94913609.7
(22) Anmeldetag: 18.04.1994
(51) Int. Cl.: C08G 65/34

(54) **VERWENDUNG VON POLYACETALEN ZUR HERSTELLUNG VON KOMPOSTIERBAREN FORMKÖRPERN**
USE OF POLYACETALS FOR PRODUCING COMPOSTABLE SHAPED BODIES
UTILISATION DE POLYACETALS POUR PRODUIRE DES CORPS MOULES COMPOSTABLES

(30) Priorität: 20.04.1993 DE 4312753
(43) Veröffentlichungstag der Anmeldung: 07.02.1996
(73) Patentinhaber: BASF AKTIENGESELLSCHAFT, 67056 Ludwigshafen (DE)
(72) Erfinder: KRONER, Matthias, D-67304 Eisenberg (DE); SCHORNICK, Gunnar, D-67271 Neuleiningen (DE); SCHUMACHER, Karl-Heinz, D-67435 Neustadt (DE); WISTUBA, Eckehardt, D-67098 Bad Duerkheim (DE)
(86) Internationale Anmeldenummer: EP9401200
(87) Internationale Veröffentlichungsnummer: WO9424189

(56) Entgegenhaltungen:
- WO-A-91/11466
- GB-A- 789 458
- US-A- 4 549 010
- US-A- 5 466 762
- ACCOUNTS OF CHEMICAL RESEARCH Bd. 26, Nr. 3 , März 1993 , WASHINGTON US Seiten 105 - 110 SWIFT,GRAHAM 'Directions for Environmentally Biodegradable Polymer Research'

## Beschreibung

Die Erfindung betrifft die Verwendung von Acetaldehydacetaleinheiten enthaltenden Polyacetalen und Mischungen dieser Polyacetale mit Füllstoffen zur Herstellung von kompostierbaren Formkörpern sowie Windeln, die als Außenlage Acetaldehydacetaleinheiten enthaltende Polyacetale in Form einer kompostierbaren Folie oder einer kompostierbaren Beschichtung aufweisen.

Thermoplastische Kunststoffe, wie Polyethylen, Polypropylen und Polystyrol, die üblicherweise zur Herstellung von Verpackungen verwendet werden, sind biologisch nicht abbaubar. Die gebrauchten Verpackungsmaterialien werden dadurch entsorgt, daß man sie einer Wiederverwendung zuführt, sie zur Energiegewinnung verbrennt oder auf Mülldeponien lagert. Aufgrund zunehmender Müllmengen gewinnt jedoch die Kompostierung von Müll immer mehr an Bedeutung. Die oben namentlich genannten Kunststoffe sind jedoch nicht kompostierbar. So können beispielsweise Windeln, die aus Außenlage eine Folie aus Polyethylen enthalten, nicht ohne weiteres kompostiert werden. Hierzu muß man zunächst die Folie vorher in einem zusätzlichen Verfahrensschritt von den übrigen Bestandteilen der Windel trennen oder die Windel mechanisch verkleinern. Bei der mechanischen Verkleinerung fallen jedoch verhältnismäßig große Folienfetzen an, die dann beim Ausbringen des Biomülls und auch später im Boden unerwünscht sind und zu Schwierigkeiten führen.

Aus Journal of Polymer Science: Polymer Letters Edition, Vol. 18, 293-297 (1980) ist die Herstellung von Polyacetalen durch Säure katalysierte Anlagerung von Polyolen an Divinylether bekannt. So erhält man beispielsweise durch Polyaddition von trans-1,4-Cyclohexandimethanol an Butandioldivinylether unter der katalytischen Wirkung von p-Toluolsulfonsäure ein Polyacetal mit einem Molekulargewicht von 200 000. Die beschriebenen Polyacetale werden in der Medizin zur kontrollierten Freisetzung von Wirkstoffen verwendet.

Aus der nicht vorveröffentlichten deutschen Patentanmeldung P 4142130.2 ist die Verwendung von Polyacetalen, die durch kationisch initiierte Polyaddition von Divinylethern und Dihydroxyverbindungen sowie gegebenenfalls Monohydroxyverbindungen erhältlich sind, als Zusatz zu phosphatarmen und phosphatfreien Wasch- und Reinigungsmitteln bekannt. Außerdem sind daraus Polyacetale bekannt, die durch kationisch initiierte Polyaddition von Divinylethern und Dihydroxyverbindungen und anschließende Addition von Monohydroxyverbindungen an 5 bis 95% der Vinylethergruppen im Polyacetal erhältlich sind, so daß die entstehenden Polyacetale Vinylethergruppen aufweisen.

Der vorliegenden Erfindung liegt die Aufgabe zugrunde, Materialien zur Herstellung von kompostierbaren oder verrottbaren Formkörpern sowie Windeln, die kompostiert werden können, zur Verfügung zu stellen.

Die Aufgabe wird erfindungsgemäß gelöst durch Verwendung von Acetaldehydacetaleinheiten enthaltenden Polyacetalen und Mischungen dieser Polyacetale mit Füllstoffen zur Herstellung von kompostierbaren Formkörpern.

Gegenstand der Erfindung sind außerdem Windeln, die als Außenlage Acetaldehydacetaleinheiten enthaltende Polyacetale in Form einer kompostierbaren Folie oder einer kompostierbaren Beschichtung aufweisen.

Vorzugsweise in Betracht kommende Acetaldehydacetaleinheiten enthaltende Polyacetale sind erhältlich durch kationisch initiierte Polyaddition von Monovinylethern von zweiwertigen Alkoholen oder durch kationisch initiierte Polyaddition von
(a) zweiwertigen Alkoholen, die gegebenenfalls bis zu 50 Mol-% durch andere Alkohole ersetzt sein können und
(b) Divinylether oder Divinylether von zweiwertigen Alkoholen, wobei die genannten Divinylether gegebenenfalls bis zu 50 Mol.-% durch andere Vinylether ersetzt sein können.

Beispielsweise entstehen durch Polyaddition von Diolen und Divinylethern Polyacetale, in denen die zugrundeliegenden Diole als Wiederholungseinheiten über Acetaldehydacetaleinheiten verbunden sind. Solche Polymeren besitzen aufgrund der Acetaldehydacetaleinheiten Sollbruchstellen, die enzymatisch oder hydrolytisch unter Molgewichtsabbau in gut biologisch abbaubare Polymerfragmente oder Diole gespalten werden können. Der enzymatische Angriff auf diese Polyacetale erfolgt durch Mikroorganismen, z.B. durch Bakterien oder durch Pilze. Der hydrolytische Abbau wird durch Absenkung des pH-Wertes des Mediums erreicht, das die Polyacetale umgibt. Bei einer pH-Wertabsenkung auf Werte unterhalb von 7, z.B. aufgrund von biologischen Prozessen im Kompost oder im Boden oder durch Zugabe von Säuren können die Polyacetale kompostiert werden bzw. verrotten. Auch die Acidität des in der Erdatmosphäre vorhandenen Kohlendioxids ist ausreichend, um eine sauer initiierte Hydrolyse der Polyacetale zu bewirken. Bei der Hydrolyse der Polyacetale entstehen im wesentlichen Acetaldehyd und Diole, die den Divinylethern der Komponente (b) zugrundeliegen sowie aus der Komponente (a) stammen. Acetaldehyd und Diole sind unter Kompostierbedingungen vollständig biologisch abbaubar.

Als Acetaldehyacetaleinheiten enthaltenden Polyacetale kommen die in den oben angegebenen Literaturstellen beschriebenen Polyacetale in Betracht, sowie vorzugsweise die Polyacetale, die in den älteren, nicht vorveröffentlichten deutschen Patentanmeldungen P 21 30 428.4, P 41 42 130.2, P 42 33 340.7 und P 42 37 337.9 beschrieben sind. Die in den älteren Anmeldungen beschriebenen Polyacetale werden im wesentlichen als Zusatz zu phosphatarmen und phosphatfreien Wasch- und Reinigungsmitteln oder in Geschirrspülmitteln verwendet. Sie sind außerdem auch als Dispergiermittel geeignet. Die bevorzugt zum Einsatz gelangenden Polyacetale sind durch kationisch initiierte Polyaddition von
(a) zweiwertigen Alkoholen, die gegebenenfalls bis zu 50 Mol-% durch andere Alkohole ersetzt sein können und
(b) Divinylether oder Divinylether von zweiwertigen Alkoholen, wobei die genannten Divinylether gegebenenfalls bis zu 50 Mol.-% durch andere Vinylether ersetzt sein können, oder durch kationisch initiierte Polyaddition von Monovinylethern von zweiwertigen Alkoholen erhältlich.

Als zweiwertige Alkohole der Komponente (a) kommen alle aliphatischen und aromatischen Diole mit mehr als 2 Kohlenstoffatomen in Betracht, z.B. Alkylenglykole und Polyalkylenglykole wie Ethylenglykol, Diethylenglykol, Propylenglykol, Dipropylenglykol, Polypropylenglykol, Polytetrahydrofuran, Butandiol, Hexandiol, Cyclohexandimethanol, Cyclohexandiol, Weinsäurediethylester, Bisphenol A, Bis(Hydroxyphenyl)sulfon, Bis(Hydroxyphenyl)keton, hydroxylierte Fettsäureester, oligomere oder polymere Ester aus Dicarbonsäuren und Diolen mit Alkoholendgruppen.

Zur Herstellung dieser Alkoholendgruppen enthaltenden oligomeren oder polymeren Ester können Dicarbonsäuren oder Hydroxydicarbonsäuren mit Diolen umgesetzt werden. Als Dicarbonsäuren eignen sich beispielsweise aromatische Dicarbonsäuren wie Terephthalsäure, Phthalsäure oder Isophthalsäure aber auch aliphatische Dicarbonsäuren wie C₂- bis C₁₀-Dicarbonsäuren, insbesondere Oxalsäure, Bernsteinsäure oder Adipinsäure. Es kommen aber auch Glykolsäure oder Milchsäure in Betracht. Als Diole können sowohl aromatische als auch aliphatische Diole verwendet werden. Besonders bevorzugt werden C₂- bis C₁₀-Alkylendiole, worunter Ethylenglykol, 1,2-Propylenglykol, 1,3-Propylenglykol, 1,2-Butandiol, 1,4-Butandiol und 1,6-Hexandiol besonders geeignet sind. Auch cycloaliphatische Diole wie Cyclohexandimethanol können verwendet werden. Als Beispiel für aromatische Diole sei Bisphenol A genannt. Selbstverständlich können auch Mischungen unterschiedlicher Dicarbonsäuren oder unterschiedlicher Diole zur Herstellung der Alkoholendgruppen enthaltenden oligomeren oder polymeren Ester eingesetzt werden.

Ganz besonders bevorzugte Alkoholendgruppen enthaltende oligomere oder polymere Ester sind aus den folgenden monomeren Einheiten aufgebaut:
Bernsteinsäure und 1,4-Butandiol,
   Bernsteinsäure und Ethylenglykol,
   Bernsteinsäure und 1,6-Hexandiol,
Adipinsäure und 1,4-Butandiol,
   Adipinsäure und Ethylenglykol,
   Adipinsäure und 1,6-Hexandiol,
Terephthalsäure und 1,4-Butandiol,
   Terephthalsäure und Ethylenglykol,
   Terephthalsäure und 1,6-Hexandiol,
Terephthalsäure, Adipinsäure und 1,4-Butandiol,
   Terephthalsäure, Adipinsäure und Ethylenglykol,
   Terephthalsäure, Adipinsäure und 1,6-Hexandiol,
Milchsäure und 1,4-Butandiol,
   Milchsäure und Ethylenglykol,
   Milchsäure und 1,6-Hexandiol,
Adipinsäure, Milchsäure und 1,4-Butandiol,
   Adipinsäure, Milchsäure und Ethylenglykol,
   Adipinsäure, Milchsäure und 1,6-Hexandiol,
Bernsteinsäure, Milchsäure und 1,4-Butandiol,
   Bernsteinsäure, Milchsäure und Ethylenglykol,
   Bernsteinsäure, Milchsäure und 1,6-Hexandiol.

Im allgemeinen weisen diese Alkoholendgruppen enthaltenden oligomeren oder polymeren Ester mittlere Polymerisationsgrade von 3 bis 10 000 auf. In der Regel haben sie OH-Zahlen von weniger als 100, bevorzugt 1 bis 70 und Säurezahlen von weniger als 20, bevorzugt weniger als 10.

Die zweiwertigen Alkohole (a) können dabei als Gemische eingesetzt werden, um statistische Polyacetale oder nacheinander, um Blockpolyacetale zu erzeugen. Es lassen sich auch Blockacetale herstellen, indem man Divinylether (b) und die zweiwertigen Alkohole (a) in einem von 1 abweichenden Molverhältnis zur Reaktion bringt und die entstehenden Blöcke, die einerseits Alkoholgruppen und andererseits Divinylethergruppen tragen, dann zur Herstellung der Blockpolyacetale vereinigt. Blockcopolyacetale dieser Art werden beispielsweise in der nicht vorveröffentlichten DE-Anmeldung P 42 37 337.9 beschrieben.

Die zweiwertigen Alkohole können gegebenenfalls bis zu 50 Mol-% durch andere Alkohole ersetzt sein. Als andere Alkohole kommen sowohl einwertige sowie dreiwertige und höherwertige Alkohole in Betracht. Acetaldehydacetaleinheiten enthaltende Polyacetale dieser Art sind beispielsweise aus der älteren DE-Anmeldung P 4 142 130.2 und der älteren DE-Anmeldung P 4 233 340.7 bekannt. Mehrwertige Alkohole können beispielsweise Trimethylolpropan, Glycerin oder Kohlenhydrate oder Polysaccharide sein, z.B. Glucose, Methylglucosid, Saccharose, Stärke und Oligofructoside wie Inulin oder Hydrierprodukte wie Erythrit oder Sorbit. Mehrwertige Alkohole können auch durch Hydroxylierung von ungesättigten Ölen und Fetten hergestellt werden. Geeignete mehrwertige Alkohole sind auch dadurch erhältlich, daß man beispielsweise Butandiolmonovinylether mit Maleinsäuredimethylester radikalisch copolymerisert.

Als Divinyletherkomponente (b) werden die Divinyletherderivate der Alkoholkomponente (a) verwendet. Beispiele sind Butandioldivinylether, Cyclohexandimethanoldivinylether oder Hexandioldivinylether.

Die Divinylether oder der Divinylether selbst können gegebenenfalls bis zu 50 Mol-% durch andere Vinylether ersetzt sein. Hierbei kommen sowohl Monovinylether und in Analogie zu den Alkohlen der Komponente (a) Vinylether von mehrwertigen Alkoholen in Betracht, deren OH-Gruppen vollständig vinyliert sind, z.B. Trivinylether von Glycerin oder von Trimethylolpropan.

Zur Herstellung der Polyacetale können auch Monovinylether der zweiwertigen Alkohole der oben beschriebenen Komponente (a) allein verwendet werden. Beispiele sind Butandiolmonovinylether, Hexandiolmonovinylether, Cyclohexandimethanolmonovinylether.

Die Herstellung der Acetaldehydacetalgruppen enthaltenen Polyacetale erfolgt vorzugsweise in Substanz, wobei man als kationischen Initiator vorzugsweise Carbonsäuren wie Oxalsäure, Weinsäure oder Zitronensäure einsetzt. Die Komponenten (a) und 5 (b) werden vorzugsweise in einem Molverhältnis von 3:2 bis 2:3 bei der kationisch initiierten Polyaddition eingesetzt. Die Acetaldehydacetaleinheiten enthaltenden Polyacetale besitzen thermoplastische Eigenschaften. Die Glasumwandlungspunkte der Polyacetale können innerhalb eines weiten Temperaturbereiches eingestellt werden, z.B. in dem Bereich von -60 bis 100°C. Sie liegen beispielsweise bei Verwendung von Cyclohexandimethanoldivinylether und aliphatischen Diolen wie Butandiol oder Hexandiol in dem Bereich von -45 bis 0°C. Solche Polyacetale sind bei Zimmertemperatur harzige oder schwerfließende klebrige Massen. Sie können zur Beschichtung oder auch als Klebstoff verwendet werden.

Polyacetale, die aus cyclischen Divinylethern und cyclischen Diolen hergestellt werden, z.B. durch kationisch initiierte Polyaddition von Cyclohexandimethanoldivinylether und Bisphenol A besitzten Glasumwandlungspunkte Tg in dem Bereich von 10 bis 50°C.

Falls man bei der Herstellung der Polyacetale die Diole zu mehr als 1 Gew.-% durch höherwertige Alkohole ersetzt, so können Polyacetale mit kautschukartigen Eigenschaften entstehen. Diese Polyacetale sind dann in Verdünnungsmitteln nicht mehr vollständig löslich, sondern nur noch quellbar. Durch Variation der Komponenten (a) und (b) können die Eigenschaften der Acetaldehydacetaleinheiten enthaltenden Polyacetale in weiten Bereichen dem jeweiligen Anwendungszweck angepaßt werden.

Den Acetaldehydacetaleinheiten enthaltenden Polyacetale haben vorzugsweise K-Werte von 9 bis 70 (bestimmt nach H. Fikentscher in 1%iger Lösung in Tetrahydrofuran bei 25°C).

Die erfindungsgemäß zu verwendenden Polyacetale können in der Schmelze, als Lösung in organischen Lösemitteln oder in Form von wäßrigen Sekundärdispersionen verarbeitet werden. Polyacetalschmelzen besitzen bei genügend hohen Temperaturen, z.B. in dem Bereich von 100 bis 200°C niedrige Viskositäten. Solche Schmelzen lassen sich gießen oder können extrudiert werden. Solche. Schmelzen eignen sich beispielsweise als Beschichtungsmittel für Papier und Papierprodukte. Unter Papierprodukten sollen beispielsweise Vliese verstanden werden. Außerdem eignen sich die erfindungsgemäß zu verwendenden Polyacetale zum Beschichten von Folien aus anderen kompostierbaren Materialien, wie Stärke oder Cellulose.

Die Acetaldehydacetaleinheiten enthaltenden Polymerisate sind in organischen Lösemitteln löslich. Solche Lösungen von Polyacetalen können beispielsweise auch zur Herstellung von Beschichtungen verwendet werden. Geeignete Lösungsmittel sind beispielsweise Essigsäureethylester, Aceton oder Tetrahydrofuran. Wäßrige Dispersionen der erfindungsgemäß zu verwendenden Polyacetale erhält man beispielsweise dadurch, daß man die Polyacetale in Wasser bei pH-Werten oberhalb von 7 dispergiert. Solche wäßrigen Sekundärdispersionen werden beispielsweise dadurch hergestellt, daß man die erfindungsgemäß zu verwendenden Polyacetale in Wasser aufschlämmt und durch Einwirkung starker Scherkräfte in dem Temperaturbereich von 0 bis 100°C dispergiert. Die Einwirkung von Ultraschall auf feinteilige Polyacetale in wäßriger Aufschlämmung erleichtert das Dispergieren der Polyacetale. Das Dispergieren wird außerdem durch den Zusatz von Tensiden, Salzen, Schutzkolloiden und/oder Emulgatoren unterstützt.

Lösungen oder Sekundärdispersionen der Acetaldehydacetaleinheiten enthaltenden Polyacetale können durch Walzen, Streichen, Spritzen oder Gießen auf Beschichtungsunterlagen aufgebracht werden. Geeignete Beschichtungsunterlagen sind bereits oben im Zusammenhang mit der Schmelzbeschichtung genannt worden. Vorzugsweise werden solche Materialien beschichtet, die kompostierbar sind oder verrotten. Solche Rohstoffe sind beispielsweise Formkörper aus Cellulose oder Stärke.

Die Acetaldehydactaleinheiten enthaltenden Polyacetale können außerdem zur Herstellung von Formkörpern verwendet werden, die kompostierbar sind. Unter Formkörpern sollen dabei alle Gegenstände verstanden werden, die zu einem einmaligen Gebrauch bestimmt sind, beispielsweise Wegwerfgegenstände, wie Geschirr, Besteck, Müllsäcke, Folien für die Landwirtschaft zur Ernteverfrühung, Verpackungsfolien und Gefäße für die Anzucht von Pflanzen. Gegenstände aus dem erfindungsgemäß zu verwendenden Polyacetalen werden in Gegenwart von Wasser und Mikroorganismen oder auch nur von Wasser allein und Zutritt von Luft abgebaut. Solche Materialien verrotten daher nach einer gewissen Zeit unter Kompostierungsbedingungen.

Die oben beschriebenen Formkörper können ggf. auch aus einer Mischung von Acetaldehydacetaleinheiten enthaltenden Polyacetalen und Füllstoffen hergestellt werden. Diese Mischungen können beispielsweise 0,5 bis 99 Gew.-% eines Füllstoffs enthalten. Geeignete Füllstoffe sind beispielsweise Ruß, Stärke, Ligninpulver, Cellulosefasern, Eisenoxide, Tonmineralien, Erze, Calciumcarbonat, Calciumsulfat, Bariumsulfat und Titandioxid. Die Füllstoffe können zum Teil auch Stabilisatoren für die Acetaldehydacetaleinheiten enthaltenden Polyacetale sein, z.B. Calciumcarbonat, Natriumcarbonat, Natriumhydrogencarbonat oder Calciumoxid. In einigen Fällen hat es sich als vorteilhaft erwiesen, die erfindungsgemäß zu verwendenden Polyacetale mit 0,1 bis 30 Gew.-% an solchen alkalischen Füllstoffen, die als Stabilisator wirksam sind, zu mischen. Die erfindungsgemäß zu verwendenden Polyacetale können außerdem durch den Zusatz von anorganischen oder organischen Farbstoffen beliebig eingefärbt werden. Die Farbstoffe können im weitesten Sinne auch als Füllstoff angesehen werden. Wenn die erfindungsgemäß zu verwendenden Polyacetale noch Vinylethergruppen enthalten, können sie mit OH-Gruppen aufweisenden Beschichtungsunterlagen, z.B. mit Stärke oder Cellulose, unter Einwirkung von Säuren kovalent verbunden werden. Mischungen aus solchen Polyacetalen und Stärke oder Cellulose sind somit nicht nur physikalisch sondern chemisch verbunden. Es tritt eine Hydrophobierung von Stärke oder Cellulose ein.

Von besonderem Interesse ist die Verwendung der Acetaldehydacetaleinheiten enthaltenden Polyacetale in Form einer kompostierbaren Folie oder einer kompostierbaren Beschichtung als Außenlage von Windeln. Die Außenlage der Windeln verhindert wirksam den Durchtritt von Urin, der im Innern der Windel vom Fluff und gegebenenfalls den Superabsorbern, z.B. auf Basis von vernetzter Polyacrylsäure oder vernetztem Polyacrylamid, absorbiert wird. Als Innenlage der Windel wird meistens ein Faservlies aus einem Cellulosematerial verwendet. Die Außenlage der beschriebenen Windeln ist biologisch abbaubar und damit kompostierbar. Sie zerfällt beim Kompostieren, so daß die gesamte Windel verrottet, während mit einer Außenlage aus Polyethylen versehene Windeln nicht ohne vorherige Zerkleinerung oder aufwendige Abtrennung der Polyethylenfolie kompostiert werden können.

### Beispiele

### Herstellung des Polyacetals 1

80,11 g eines Isomerengemisches von Cyclohexandimethanoldivinylether werden unter Rühren mit 290 mg wasserfreier Oxalsäure versetzt und auf eine Temperatur von 45°C erwärmt. Nachdem die Oxalsäure vollständig gelöst ist, wird die Temperatur der Mischung auf 110°C erhöht. Bei dieser Temperatur gibt man dann innerhalb von 1 Stunde 93,17 g Bisphenol A (2,2-Bis-(4-hydroxyphenyl)propan) portionsweise zu. Danach wird die Reaktionsmischung noch 72 Stunden bei 90°C gerührt, wobei sie polymerisiert. Danach leitet man gasförmiges Ammoniak in die Polyacetalschmelze, um die als Initiator verwendetete Oxalsäure zu neutralisieren.

Zur Herstellung von Beschichtungen stellt man eine 30%ige Lösung des Polyacetals in Tetrahydrofuran her.

### Beispiel

Ein im Haushalt übliches Vliespapier der Dicke 0,1 mm wird durch kurzes Tauchen in die oben beschriebene Polyacetallösung beidseitig beschichtet und an der Luft getrocknet. Der Anteil des Polyacetals im beschichteten Vlies beträgt 20 Gew.-%.

Das beschichtete Vlies wurde anschließend für 16 Stunden in Wasser, dessen pH-Wert auf 8 eingestellt war, gelagert und im Zugversuch nach DIN 53455-3 untersucht. Die Breite des Prüfkörpers betrug 4 mm. Die dabei erhaltenen Werte für die Zugfestigkeit und Reißfestigkeit sind in der Tabelle angegeben.

### Vergleichsbeispiel

Ein unbeschichtetes Vliespapier der Dicke 0,1 mm, das im Beispiel beschrieben ist, wird 16 Stunden in Wasser, dessen pH-Wert auf 8 eingestellt war, gelagert und anschließend im Zugversuch nach DIN 53455-3 untersucht. Die dabei für Zug- und Reißfestigkeit erhaltenen Ergebnisse sind in der Tabelle angegeben.

**Tabelle**

| | Zugfestigkeit (N/mn²) | Reißfestigkeit (N/mm²) |
|---|---|---|
| Vergleichsbeispiel | 0,34 | 0,24 |
| Beispiel | 5,54 | 5,50 |

Wie aus der Tabelle ersichtlich ist, kann sowohl die Naßzugfestigkeit als auch die Naßreißfestigkeit des Vlieses durch die Beschichtung mit einem Acetaldehydacetaleinheiten enthaltendem Polyacetal erheblich verbessert werden. Die beschichteten Vliese sind wasserdicht, d.h. sie lassen auch bei längerer Einwirkung Wasser nicht durch und werden von Wasser nicht angegriffen. Diese Eigenschaft von beschichteten Vliesen ist für die Verwendung solcher Produkte als Außenlage von Windeln von Interesse.

## Patentansprüche

1. Verwendung von Acetaldehydacetaleinheiten enthaltenden Polyacetalen und Mischungen dieser Polyacetale mit Füllstoffen zur Herstellung von kompostierbaren Formkörpern.

2. Verwendung von Acetaldehydacetaleinheiten enthaltenden Polyacetalen, erhältlich durch kationisch initiierte Polyaddition von Monovinylethern von zweiwertigen Alkoholen oder durch kationisch initiierte Polyaddition
von
(a) zweiwertigen Alkoholen, die gegebenenfalls bis zu 50 Mol-% durch andere Alkohole ersetzt sein können und
(b) Divinylether oder Divinylethern von zweiwertigen Alkoholen, wobei die genannten Divinylether gegebenenfalls bis zu 50 Mol.-% durch andere Vinylether ersetzt sein können
oder Mischungen dieser Polyacetale mit Füllstoffen zur Herstellung von kompostierbaren Formkörpern.

3. Windeln, dadurch gekennzeichnet, daß sie als Außenlage Acetaldehydacetaleinheiten enthaltende Polyacetale in Form einer kompostierbaren Folie oder einer kompostierbaren Beschichtung aufweisen.

## Claims

1. The use of polyacetals containing acetaldehyde acetal units, and of mixtures of these polyacetals with fillers, for preparing compostable moldings.

2. The use of polyacetals which contain acetaldehyde acetal units and are obtainable by cationically initiated polyaddition of monovinyl ethers of dihydric alcohols or by cationically initiated polyaddition of
(a) dihydric alcohols, which may optionally be replaced by up to 50 mol% of other alcohols, and
(b) divinyl ether or divinyl ethers of dihydric alcohols, in which case the divinyl ethers mentioned may be optionally replaced by up to 50 mol% of other vinyl ethers,
or mixtures of these polyacetals with fillers, for preparing compostable moldings.

3. Diapers with an outer layer comprising polyacetals containing acetaldehyde acetal units in the form of a compostable film or a compostable coating.

## Revendications

1. Utilisation de polyacétals contenant des motifs acétaldéhyde-acétal et de mélanges de ces polyacétals avec des matières de charges pour la préparation de corps moulés compostables.

2. Utilisation de polyacétals contenant des motifs acétaldéhyde-acétal, pouvant être obtenus par polyaddition amorcée de façon cationique d'éthers monovinyliques d'alcools bifonctionnels ou par polyaddition amorcée de façon cationique
(a) d'alcools bifonctionnels, pouvant éventuellement être remplacés par d'autres alcools jusqu'à 50% en moles et
(b) d'éther divinylique ou d'éthers divinyliques d'alcools bifonctionnels, les éthers divinyliques cités pouvant éventuellement être remplacés par d'autres éthers vinyliques jusqu'à 50% en moles,
ou des mélanges de ces polyacétals avec des matières de charge pour la préparation de corps moulés compostables.

3. Couches caractérisés en ce qu'elles présentent, en tant que revêtement extérieur, des polyacétals contenant des motifs acétaldéhyde-acétal, sous forme d'une feuille compostable ou d'un revêtement compostable.
